**Europäisches Patentamt**

**European Patent Office**

**Office européen des brevets**

(19)

(11) Publication number: **0 105 554**

**A1**

(12)

# EUROPEAN PATENT APPLICATION

(21) Application number: **83201364.3**

(22) Date of filing: **23.09.83**

(51) Int. Cl.³: **C 12 N 15/00**
**C 12 P 21/02, C 07 C 103/52**
**C 07 H 21/04, C 12 N 1/20**
**//C12R1/19**

(30) Priority: **24.09.82 NL 8203716**

(43) Date of publication of application:
**18.04.84 Bulletin 84/16**

(84) Designated Contracting States:
**AT BE CH DE FR GB IT LI LU NL SE**

(71) Applicant: **NEDERLANDSE CENTRALE ORGANISATIE**
**VOOR TOEGEPAST-NATUURWETENSCHAPPELIJK**
**ONDERZOEK**
**Juliana van Stolberglaan 148**
**NL-2595 CL The Hague(NL)**

(72) Inventor: **Pouwels, Pieter Hendrik**
**Delftweg 14**
**NL-2289 AJ Rijswijk(NL)**

(72) Inventor: **Van den Hondel, Cornelis A. M. J. J.**
**R. Burtonlaan 6**
**NL-2803 EW Gouda(NL)**

(72) Inventor: **Kos, Antonides**
**Molenzicht 30**
**NL-2317 RL Leiden(NL)**

(72) Inventor: **Nijkamp, Hendrik Johannes Jacobus**
**Prof. Bronkhorstlaan 23**
**NL-3451 ER Vleuten(NL)**

(72) Inventor: **Veltkamp, Eduard**
**Nieuwe Bussummerweg 133**
**NL-1272 CG Huizen(NL)**

(74) Representative: **Urbanus, Henricus Maria, Ir. et al,**
**c/o Vereenigde Octrooibureaux Nieuwe Parklaan 107**
**NL-2587 BP 's-Gravenhage(NL)**

(54) DNA and plasmids.

(57) The invention provides DNA, a DNA fragment or a plasmid that is characterized by containing a temperature-sensitive replication origin and a strong, regulatable promotor, such as the new plasmid pMBL24. The invention also relates to bacteria containing such a plasmid, to a process for producing proteins using such bacteria, and to a method of making the new plasmid pMBL24. The invention renders it possible to express a foreign gene in the selected bacteria in an efficient manner.

**EP 0 105 554 A1**

Title: DNA and plasmids.

This invention relates to a method of producing prokaryotic and eukaryotic proteins in bacteria in a highly efficient manner. The invention provides Escherichia coli bacteria (in particular E.coli K12 JA221) containing a plasmid (preferably the new pMBL24) capable of controlled replication, and enabling controlled and efficient expression of genes cloned in the plasmid.

The efficiency with which a cloned gene is expressed in bacteria, in particular Escherichia coli, depends, among other factors, on the number of copies of this gene that is present per bacterium (copy number). With regard to many genes, expression increases with increasing copy number. When there is a possibility of varying the number of gene copies in a controlled manner, this provides, in principle, the possibility of affecting the expression of the cloned gene in a controlled manner. The controlled variation of copy number is possible when the gene in question is part of a DNA molecule, for example, a plasmid, whose replication frequency can be affected. Uhlin et al., Gene 6 91 (1979) and Andreoli et al., J.Bacteriol.135, 612 (1978) have described plasmids whose replication frequency can be altered by changing the temperature at which the bacteria are grown. The possibility of varying the replication frequency depends on the structure of the origin of replication (ori). Of the plasmid CloDF13, mutant plasmids have been isolated (Andreoli et al.,·J. Bacteriol. 135, 612 (1978) whose replication frequency depends on the temperature of the culture medium. In the case of these plasmids, the temperature-dependent replication frequency is to be attributed to a mutation in the ori area (Stuitje et al., Nature 290, 264 (1981)). Such a temperature-sensitive replication origin is designated by ts-ori.

In order for a foreign gene to be expressed in E.coli in an efficient manner, it is necessary for such a gene to be preceded by a strong promoter. If it is also desired that expression can be regulated, it is necessary to use a promoter that can be regulated. The promoter of the tryptophan (trp) operon of E. coli is an example of a strong promoter that can also be regulated by the choice of the culture medium (Bertrand et al., Science 189 22 (1975)).

. The present invention provides a DNA, DNA fragment or plasmid that is characterized by containing a temperature-sensitive replication origin and a strong, regulatable promoter.

Preferably, according to the invention, it contains a temperature-sensitive replication origin of the plasmid CloDF13 and also the promoter of the tryptophan operon of E. coli.

In a concrete preferred embodiment the invention provides a new plasmid pMBL24 containing the ts-ori of the plasmid pVU208 and the trp promoter-operator complex of E. coli. The ts-ori serves for the controllable replication of the plasmid, and the trp promoter-operator complex for expressing cloned genes in an efficient and controllable manner.

The invention further extends to bacteria, in particular Escherichia coli bacteria containing one or more plasmids according to the invention, and also to a method of producing proteins in such bacteria. The invention also provides a method of making the new plasmid pMBL24.

The plasmid pMBL24 can be made as follows:

Starting from the plasmids already described, pVU208 (Hakkaart et al., MGG 183 326 (1981)) and pBR322 (Bolivar et al., Gene 2 95 (1977)) the plasmid pMBL21 is produced by cutting the two plasmids with restriction enzymes, in particular PvuII and PstI, and ligation by means of DNA ligase, in particular T4 DNA ligase, of the fragment of pVU208 containing the ts-ori to the fragment of pBR322 containing the tetracycline-resistant (tet) gene.

From the plasmid pMBL21 the plasmid pMBL24 is made by cutting pMBL21 by means of restriction enzymes, in particular EcoRI and PstI and ligation by means of DNA ligase, in particular T4 DNA ligase of the fragment containing the ts-ori to a fragment of pHP16 containing the trp promoter (trp P) of E. coli. The resulting plasmid contains between the trp promoter-operator complex and the ts-ori a transcription terminator which prevents that transcription from the trp promoter continues and transcribes the ts-ori. This is of importance in connection with adverse effects thereof on the replication of the plasmid (Remaut et al., Gene 15 81 (1981)).

The fragment containing the trp promoter is produced by cutting pHP16 with a restriction enzyme, in particular PvuII, ligation at the extremities with DNA ligase, in particular T4 DNA ligase, of a synthetic DNA fragment (PstI left) containing a PstI recognition sequence, and cutting the product with restriction enzymes, in particular PstI and EcoRI. The plasmid pHP16 used for this purpose is known (Enger et al., Nucl. Acid. Res. 9 1973 (1981)).

The invention also relates to this method of making pMBL24.

The invention further provides a process for producing proteins in bacteria, more specifically E. coli bacteria, in particular E.coli K12 JA221 bacteria. According to the invention, for this purpose bacteria are used which contain one or more identical plasmids with a ts-ori, in which the gene or genes whose expression is desired is, or are, under the control of the trp promoter according to the invention. In a preferred embodiment, use is made of bacteria, for example E. coli K12 JA221 bacteria, in which the expression of genes which are located on a plasmid and are under the control of the trp promoter is substantially repressed if the correct culture medium is selected. The bacterium E. coli K12 JA221 is described by Clarke and Carbon (J. Mol. Biol. 120, 517 (1978)).

According to the invention the bacteria are grown at a suitable temperature in a suitable culture medium. A suitable culture medium is Luria broth (LB culture medium). A suitable temperature for efficient expression is about 42°C. If, on the other hand, low expression is desired, a culture temperature of about 30°C is preferred.

The invention will be described in more detail with reference to the accompanying drawings and in the following examples. In said drawings, which are not to scale,

Fig. 1 shows the construction of the plasmid pMBL21 from the plasmids pVU208 and pBR322;

Fig. 2 shows the construction of the plasmid pMBL24 from the plasmids pMBL21 and pHP16;

Fig. 3 shows the construction of the plasmid pMBL23 from the plasmids pHP16 and pBR322;

Fig. 4 shows an electrophoresis pattern of DNA, from E. coli K12 JA221 bacteria with the plasmid pMBL23 or pMBL24 therein, cultured at 30° or 42°C.

Example I

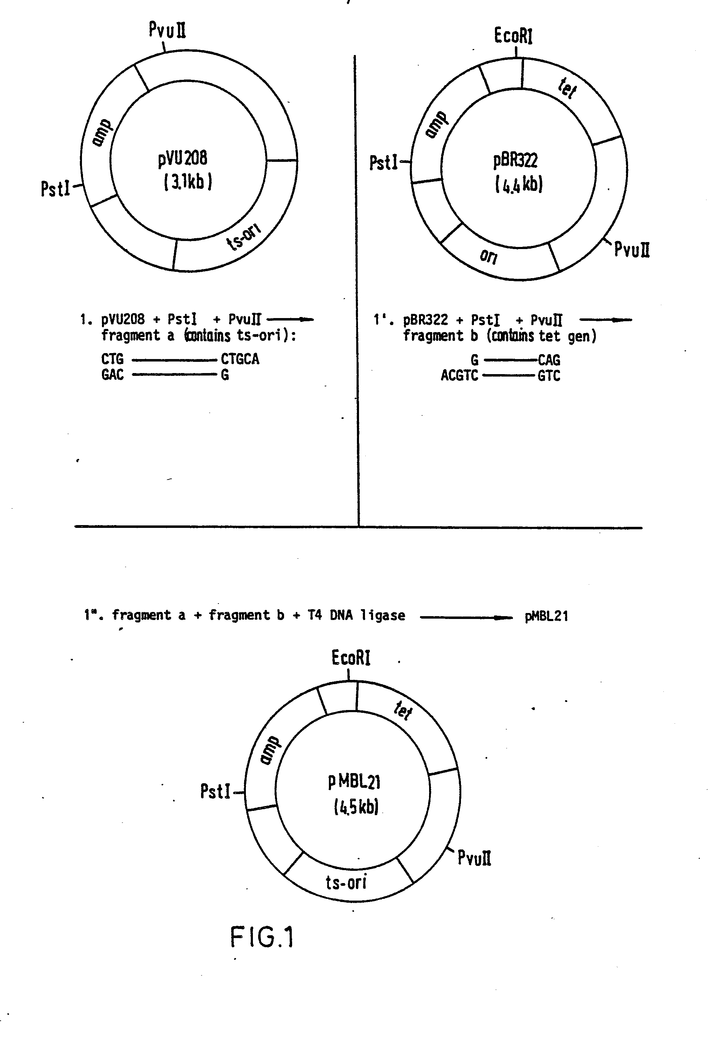

Construction of the plasmid pMBL21 (Fig. 1): For the construction of a plasmid with the ts-ori, the plasmid pBR322 is used as the starting material. The replication origin of pBR322 was replaced by a thermosensitive replication origin of plasmid pVU208, in which the ts-ori originates from C10DF13.

For this purpose, the plasmid pBR322 was cut with PstI and PvuII, and the fragment containing the tet gene was isolated. From plasmid pVI208, after cutting with PstI and PvuII, the fragment containing the ts-ori was isolated. The two fragments were ligated by means of T4 DNA ligase. By ligating the two fragments, the gene for ampicillin resistance (amp) is repaired. After the transformation of E.coli JA221 bacteria, selection of transformed bacteria was effected by plating out on nutrients to which ampicillin had been added. The formed plasmid pMBL21 contains an amp gene, a tet gene and a ts-ori.

Example II

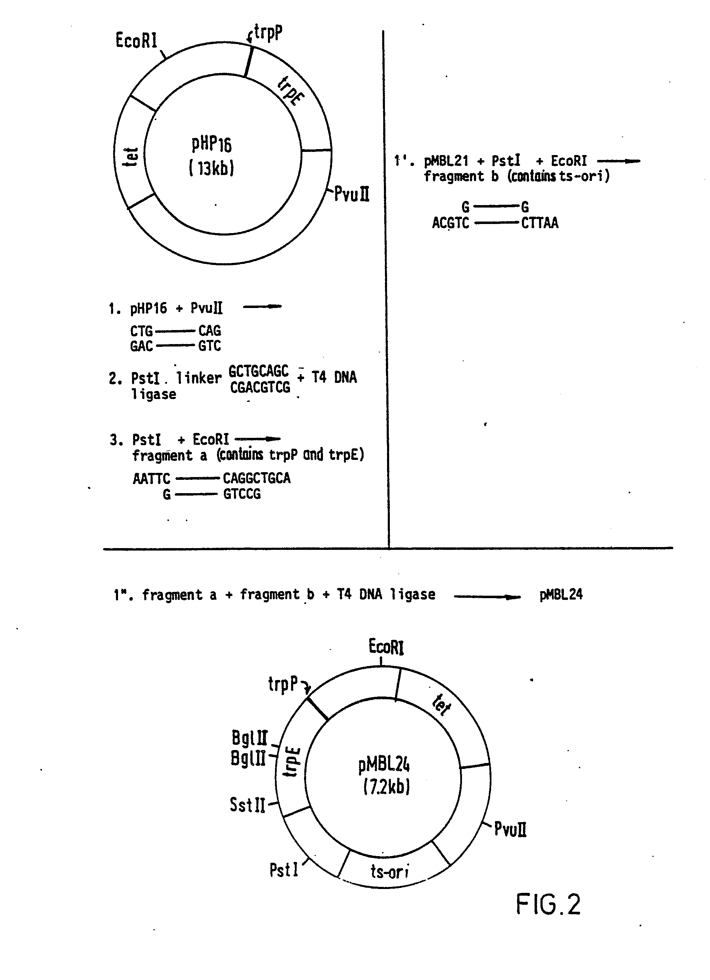

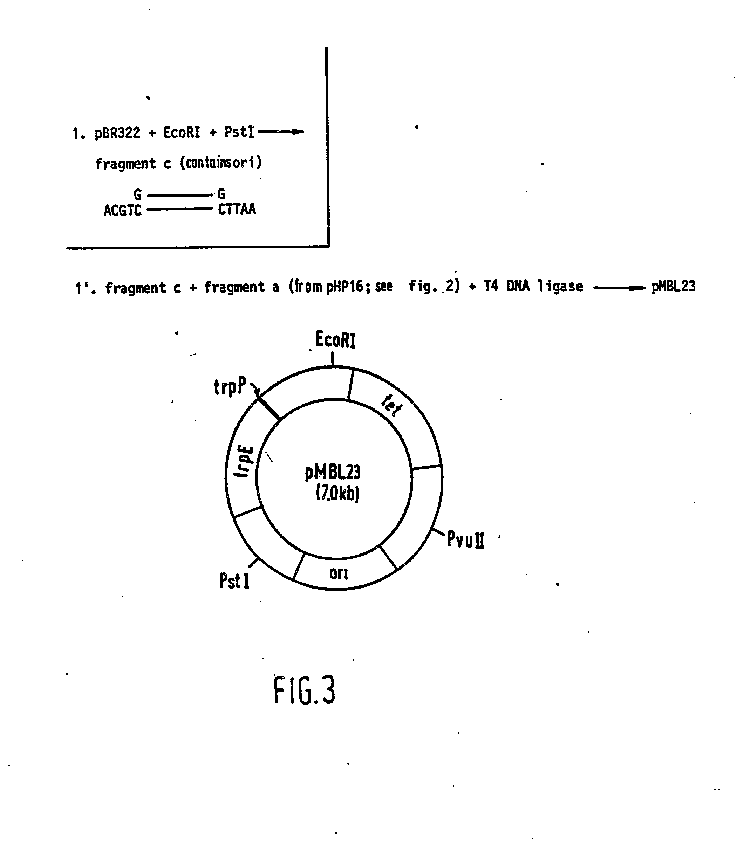

The construction of the plasmids pMBL24 and pMBL23 (Figs. 2 and 3): For the construction of pMBL24 (Fig. 2) pMBL21 was cut with EcoRI and PstI, and the fragment containing the ts-ori was isolated. The fragment containing the trp promoter-operator complex and trpE was isolated, after cutting of pHP16 with PvuII, ligation at the extremities by means of T4 DNA ligase of a synthetic DNA fragment

GCTGCAGC
CGACGTCG containing a recognition site for the restriction enzyme

PstI, and after cutting of the formed product with PstI en EcoRI.

These fragments were ligated by means of T4 DNA ligase. After the transformation of E.coli K12 trpE bacteria, the selection of transformed bacteria was effected by plating on nutrients containing tetracycline but notryptophan. The structure of the formed plasmid pMBL24 was confirmed by means of restriction enzyme analysis. Plasmid pMBL24 contains recognition sites for the restriction enzymes BglII, SstII and PstI, which are suitable for cloning foreign genes under the control of the trp promoter.

The plasmid pMBL23 (Fig. 3), like pMBL24, contains the tet gene, the trp promoter-operator complex, and trpE, but, unlike pMBL24, has the replication origin of ColEI (Hershfield et al., Proc.Natl. Acad Sci USA 71 3455 (1974)), isolated from pBR322. Plasmid pMBL23 was produced in a manner similar to that described for pMBL24.

## Example III

Comparison of the amount of plasmid DNA and the expression efficiency of bacteria containing the plasmid pMBL24 according to the invention with those of bacteria containing the plasmid pMBL23.

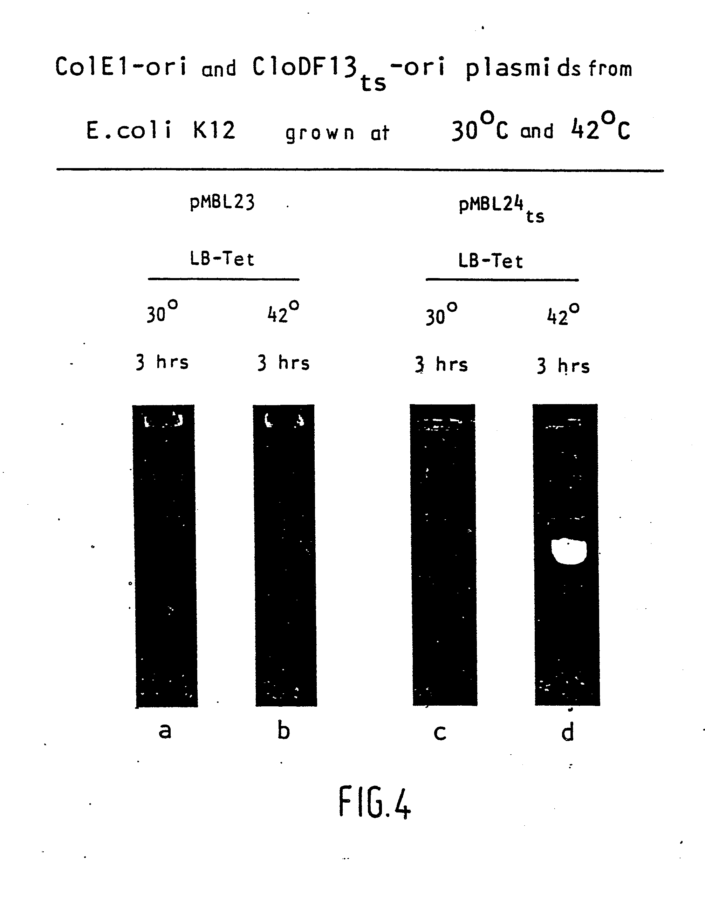

The amount of plasmid DNA was determined for E.coli K12 JA221 bacteria containing pMBL24 or pMBL23 and cultured at 30°C or 42°C. The results of this analysis are shown in Fig. 4. This figure shows the electrophoresis pattern of pMBL24 and pMBL23 DNA. Plasmid pMBL23 DNA (a,b) and pMBL24 DNA (c,d), isolated from an equal number of E.coli K12 JA221 bacteria cultured at 30°C (a,c) or 42°C (b,d), were analysed by means of gel electrophoresis in 0.9% agarose, as described by Aay and Borst, B.B.A. 269 192 (1972). The DNA in the gel was rendered visible by coloration with ethidium bromide. The amount of plasmid DNA in the gel was determined by quantitative measurement of the colour intensity. Quantitative measurements have shown that the amount of pMBL24 DNA, on average, increases by a factor 10 when the temperature of the bacterial culture is increased from 30° to 42°C, and the bacteria are subsequently grown at this temperature for 3 hours. For bacteria containing pMBL23, no difference was found in the amount of plasmid DNA when the temperature of the bacterial culture was increased from 30°C to 42°C. In both cases the amount of pMBL23 plasmid DNA was virtually equal to the amount of pMBL24 DNA synthesized at 30°C.

The effect of the temperature on the efficiency of expression of a gene under the control of the trp promoter-operator complex in pMBL24 or pMBL23 was determined by measuring the amount of the product of the trpE gene (Antranilate synthetase, ASase component I) in bacteria containing pMBL24 or pMBL23. The results, summarized in a table (3rd - 4th column) show that E.coli K12 trpE JA221 bacteria containing pMBL24 and cultured in LB culture medium (see J.H.Miller, Experiments in Molecular Genetics, Cold Spring Harbour Laboratory, 1972) at 30°C contain very small quantities of ASase. This applies to both bacteria cultured in a medium with tetracycline and to bacteria cultured in a medium without tetracycline. The amounts of ASase are comparable to those made by pMBL23 under the same conditions.

When the culturing temperature of the bacteria is increased to 42°C, the amount of ASase formed by pMBL24 containing bacteria, on average, increases 50 times. An increase of the temperature has virtually no effect on the synthesis of ASase in bacteria containing pMBL23.

An ASase activity of 1 unit (U) per mg protein means that 1% of the total amount of protein is ASase (Ito et al., J.Bacteriol 97 725 (1969)). The above results show that, in E.coli K12 JA221 bacteria, cultured at 42°C for 3 hours, the amount of ASase increases to a maximum of 28% of the total protein. In bacteria cultured at 30°C, the proportion of ASase is about 0.5%.

The above shows that the expression of a gene in E.coli K12 JA221 bacteria, under the control of the trp promoter-operator complex of pMBL24 is very low when the bacteria are cultured in LB culture medium at 30°C. Very high levels of expression can be obtained, however, when the bacteria containing pMBL24 are cultured in LB culture medium at 42°C.

Temperature increase causes a substantially higher increase of the ASase synthesis (about 50 times) than of the plasmid DNA synthesis (about 10 times) in bacteria containing pMBL24. This shows that the highly efficient gene expression at 42°C is owing to the presence on plasmid pMBL24 of both a ts-ori and a trp promoter-operator complex.

The very low efficiency of expression at low temperature in E.coli K12 bacteria and the highly efficient expression after an increase in temperature provide advantages in the cloning of genes whose product has an adverse effect on the metabolism of the bacterium. When such a gene is cloned in pMBL24 and brought under the control of the trp promoter, it can be expected that the bacterium can be cultured at 30°C without any adverse effect on the metabolism of the bacterium. By increasing the culture temperature to 42°C, the product of the gene can subsequently be synthesized in large quantities.

T A B L E

| temp. (°C) | culture medium | ASase activity (U/mg protein) pMBL24 | pMBL23 |
|---|---|---|---|
| 30 | LB | 0.5 | 0.3 |
| 42 | LB | 13 | 0.1 |
| 30 | LB + 10 µg Tet/ml | 0.7 | 0.3 |
| 42 | LB + 10 µg Tet/ml | 21 | 0.1 |
| 30 | LB + 20 µg Tet/ml | 0.3 | 0.5 |
| 42 | LB + 20 µg Tet/ml | 28 | 0.1 |

The effect of temperature increase on the expression of a gene controlled by the trp promoter was determined by measuring the trpE enzyme activity. E.coli K12 JA221 bacteria were cultured in LB culture medium in the presence or absence of tetracycline, as indicated. When the bacteria had been cultured at the temperatures indicated for 3 hours, the ASase activity was determined according to J.Ito and C.Yanofsky (J.Bacteriol. 97 725 1969)).

C L A I M S :

1. DNA, DNA fragment or plasmid,
characterized in that it contains a temperature-sensitive replication origin and a strong, regulatable promoter.

2. DNA, DNA fragment or plasmid according to claim 1, characterized in that it contains a temperature-sensitive replication origin of the plasmid CloDF13.

3. DNA, DNA fragment or plasmid according to claim 1 or 2, characterized in that it contains the promoter of the tryptophan operon of E.coli.

4. DNA, DNA fragment or plasmid according to claims 2-3, characterized in that it contains the temperature-sensitive replication origin of the plasmid pVI208 and the promoter of the tryptophan operon of the plasmid pHP16.

5. Plasmid pMBL24.

6. Bacteria, in particular Escherichia coli bacteria, containing one or more plasmids according to any of claims 1-5.

7. A process for producing proteins in bacteria, in particular Escherichia coli bacteria,
characterized by culturing bacteria containing one or more plasmids according to any of claims 1-5, in which the gene or the genes whose expression is desired is or are under the control of the strong, regulatable promoter, and at a suitable temperature in a suitable culture medium.

8. A process according to claim 7,
characterized by culturing Escherichia coli bacteria containing one or a plurality of plasmids according to claims 1-5, in which the gene or the genes whose expression is desired is or are under the control of the promoter of the tryptophan operon of E.coli, in Luria broth at a temperature of about 30°C so long as no expression is desired, and at a temperature of about 42°C when high expression is desired.

9. A method of making plasmid pMBL24, characterized by cutting the plasmids pVU208 and pBR322 with restriction enzymes, in particular PstI and PvuII, ligating a fragment originating from pVU208 and containing the temperature sensitive origin, by means of DNA ligase, in particular T4 DNA ligase, to a fragment from pBR322 which contains the tetracycline resistance gene to form a plasmid pMBL21, isolating therefrom, by cutting with restriction enzymes, in particular PstI and EcoRI, a fragment containing the temperature-sensitive origin, ligating said fragment by means of DNA ligase, in particular T4 DNA ligase, to a fragment from pHP16 which contains the promoter of the tryptophan operon, produced by cutting pHP16 with a restriction enzyme, in particular PvuII, ligating between the extremities a synthetic DNA fragment (left) with the nucleotides sequence $\frac{\text{GCTGCAGC}}{\text{CGACGTCG}}$ by means of DNA ligase, in particular T4DNA ligase, and cutting the product with restriction enzymes, in particular PstI and EcoRI.

FIG.1

EcoRI     trpP

trpE

tet

pHP16
(13kb)

PvuII

1'. pMBL21 + PstI + EcoRI ⟶
fragment b (contains ts-ori)

G ———— G
ACGTC ————— CTTAA

1. pHP16 + PvuII ⟶

CTG ———— CAG
GAC ———— GTC

2. PstI . linker $\frac{GCTGCAGC}{CGACGTCG}$ + T4 DNA ligase

3. PstI + EcoRI ⟶
fragment a (contains trpP and trpE)

AATTC ———— CAGGCTGCA
   G ———— GTCCG

1". fragment a + fragment b + T4 DNA ligase ⟶ pMBL24

EcoRI

trpP

tet

BglII
BglII

trpE

pMBL24
(7.2kb)

SstII

PstI    ts-ori

PvuII

FIG.2

1. pBR322 + EcoRI + PstI ⟶

    fragment c (contains ori)

```
      G ——————— G
  ACGTC ——————— CTTAA
```

1'. fragment c + fragment a (from pHP16; see fig. 2) + T4 DNA ligase ⟶ pMBL23

FIG.3

ColE1-ori and CloDF13$_{ts}$-ori plasmids from

E.coli K12    grown at    30$^o$C and 42$^o$C

| pMBL23 | | pMBL24$_{ts}$ | |
| --- | --- | --- | --- |
| LB-Tet | | LB-Tet | |
| 30$^o$ | 42$^o$ | 30$^o$ | 42$^o$ |
| 3 hrs | 3 hrs | 3 hrs | 3 hrs |

| a | b | c | d |

FIG.4

## EUROPEAN SEARCH REPORT

European Patent
Office

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl. ³) |
| X | ANNALS OF THE NEW YORK ACADEMY OF SCIENCES, vol. 369, 1981 TADAYUKI IMANAKA et al.:"A perspective on the application of genetic engineering: Stability of recombinant plasmid", pages 1-14 * Pages 1-5; page 12, last paragraph; page 14, conclusion * | 1,3 | C 12 N 15/00 C 12 P 21/02 C 07 C 103/52 C 07 H 21/04 C 12 N 1/20 // C 12 R 1/19 |
| A | WO-A-8 202 901 (CETUS CORPORATION) * Claims 1-14 * | 1 | |
| A | CHEMICAL ABSTRACTS, vol. 89, no. 19, November 6, 1978, page 283, abstract no. 159981e COLUMBUS, OHIO (US) P.M. ANDREOLI et al.:"Isolation and characterization of a Clo DF13::Tn901 plasmid mutant with thermosensitive control of DNA replication". & J. Bacteriol. 1978, 135(2), 612-21 | 1 | TECHNICAL FIELDS SEARCHED (Int. Cl. ³)  C 12 N |
| A | NUCLEIC ACIDS RESEARCH, vol. 9, no. 8, 1981, IRL Press LTD, London (GB) B.E. ENGER-VALK et al.:"The construction of new vehicles for the cloning of transcription termination signals", pages 1973-1989 * The whole document * | 1 | |

--- -/-

The present search report has been drawn up for all claims

| Place of search THE HAGUE | Date of completion of the search 16-12-1983 | Examiner DELANGHE L.L.M. |
|---|---|---|

## EUROPEAN SEARCH REPORT

**European Patent Office**

EP 83 20 1364

| DOCUMENTS CONSIDERED TO BE RELEVANT | | | Page 2 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A | EP-A-0 003 062 (GUSTAFSSON) <br> * Claims 1-10 * <br><br> ----- | 1 | |

TECHNICAL FIELDS
SEARCHED (Int. Cl. ³)

The present search report has been drawn up for all claims

| Place of search | Date of completion of the search | Examiner |
|---|---|---|
| THE HAGUE | 16-12-1983 | DELANGHE L.L.M. |